# EUROPÄISCHE PATENTSCHRIFT

(11) **EP 1 046 637 B1**
(45) Veröffentlichungstag und Bekanntmachung des Hinweises auf die Patenterteilung: **14.01.2004**
(21) Anmeldenummer: 00105352.9
(22) Anmeldetag: 17.03.2000
(51) Int. Cl.: C07C 263/20

(54) **Verfahren zur Absenkung des Chlorgehaltes von niedermolekularen Isocyanaten**
Process for the reduction of chlorine content of low molecular weight isocyanates
Procédé de réduction de la teneur en chlore d'isocyanates à bas poids moléculaire

(30) Priorität: 30.03.1999 DE 19914291
(43) Veröffentlichungstag der Anmeldung: 25.10.2000
(73) Patentinhaber: BAYER AG, 51368 Leverkusen (DE)
(72) Erfinder: Danielmeier, Karsten, Dr., Bethel Park, PA 15102 (US); Mager, Dieter, 51373 Leverkusen (DE); Halpaap, Reinhard, Dr., 51519 Odenthal (DE); Brahm, Martin, Dr., 51519 Odenthal (DE); Hoffman, Eric, Baytown, TX 77520 (US)

(56) Entgegenhaltungen:
- EP-A- 0 445 602
- DD-A- 288 596
- DE-A- 4 006 978

## Beschreibung

Die Erfindung betrifft ein neues Verfahren zur Reinigung organischer Isocyanate oder Isocyanatgemische von Chlorverbindungen durch Behandlung mit hochmolekularen Phosphorsäureestern. Die so gereinigten Isocyanate eignen sich vorzugsweise zur Verwendung in Beschichtungen, Polyurethanformteilen und als Zwischenprodukte.

Herstellbedingte Verunreinigungen wechselnder Art und Menge in organischen Isocyanaten sind Anlaß für Aktivitätsschwankungen. Solche Aktivitätsschwankungen sind ungünstig für eine reproduzierbare, das heißt wirtschaftliche, Verwendung. So enthalten sowohl aromatische Isocyanate, zum Beispiel die bekannten Phosgenierungsprodukte von Anilin-Formaldehyd-Kondensaten oder auch 2,4- und 2,6-Disocyanatotoluol, aber auch aliphatische Isocyanate, wie zum Beispiel Isophorondiisocyanat, eine Fülle derartiger Verunreinigungen. Es handelt sich hierbei vor allem um Chlor-enthaltende Verbindungen, die immer dann Aktivitätsschwankungen verursachen, wenn es sich um "leicht bewegliches", sogenanntes hydrolisierbares Chlor handelt. Ein Teil dieser Verbindungen erweist sich als relativ stabil und verbleibt auch nach Destillation in den Isocyanaten. Er beeinflußt neben der Aktivität auch die Stabilität der Isocyanate ungünstig. Ein einheitlicher, niedriger Anteil dieser Verunreinigungen mit der Folge einer Aktivitätsnormierung und leichteren Weiterverarbeitung der Isocyanate ist daher sowohl von technischer wie auch von wirtschaftlicher Bedeutung.

Es hat daher nicht an Versuchen gefehlt, Möglichkeiten zur Entfernung von chlorhaltigen Verbindungen zu finden. In einer Vielzahl von Patentanmeldungen werden thermische Verfahren beschrieben. Beispielsweise ist bekannt, daß Erhitzen von Isocyanaten, insbesondere bei gleichzeitigem Strippen mit Inertgas, oder Erhitzen in inerten Lösungsmitteln unter Druck, bei gleichzeitigem Absaugen der flüchtigen Verbindungen, den Gehalt an leichtspaltbaren Chlorverbindungen vermindert (z.B. DE-A 12 70 036, DD 271 820, US-A 3 219 678, GB-A 1 080 717, DE-A 22 37 552; US-A 3 857 871, US-A 1 458 223, JP 0 727 808 8 A2, JP 0 634 570 7 A2, GB-A 1 384 065).

Aus den Publikationen JP 6 116 125 0 A, JP 0 516 323 1A, DE-A 19 50 101, DE-A 19 38 384, DE-A 25 32 722, DE-A 26 31 168, US-A 3 853 936, FR-A 1 555 517, DE-A 29 33 601, US-A 3 549 504 ist bekannt, daß Isocyanate durch spezielle Destillations- und Kristallisationstechniken gereinigt werden können.

Eine effektive Abtrennung der störenden Chlorverbindungen ist bei all diesen Verfahren, die auf einer rein physikalischen Behandlung beruhen, nicht gegeben, da hierdurch nur leicht spaltbare Chlorverbindungen abgetrennt werden können. Die Anwendung derartiger Verfahren beschränkt sich so auf spezielle im allgemeinen thermostabile Isocyanatverbindungen, bei deren Einsatz eine eingeschränkte Absenkung des Chlorgehaltes ausreicht.

Neben der rein thermischen Behandlung von Isocyanatverbindungen sind auch Behandlungen mit Zusätzen beschrieben, die eine verbesserte Abtrennung von störenden Chlorverbindungen erlauben. So werden in den Patentschriften JP 4 501 032 9 B, JP 4 200 413 7 B, JP 5 908 845 2 A, JP 5 910 875 3 A, JP 5 917 245 0 A, US-A 3 373 182, GB-A 1 111 581, US-A 3 759 971, US-A 4 094 894, ZA-A 8 100 606, DE-A 11 38 040, DE-A 12 86 025, US-A 3 458 558, US-A 3 264 336, SU-A 8 066 77 und DE-A 22 10 607 auf Metallen oder Alkalimetallen basierte Zusätze beschrieben, wie z.B. Metalloxide, Metallcyanamide, Metallhydride, Metallfettsäureester in Gegenwart von sterisch gehinderten Phenolen, Metallnaphthenate, Metallsilikate, Alkalimetallcarbonate oder Organometallverbindungen. Diese Zusätze können jedoch schlecht vom gereinigten Isocyanat abgetrennt werden und führen zu einer unerwünschten Metall/Metallionen-Kontamination entsprechender Isocyanatprodukte. Des weiteren bewirken fast alle Metalle und Metallkomplexe eine erhöhte Nebenproduktbildung (Trimerisat-, Carbodiimid, Dimerbildung).

Ähnliche Beschränkungen findet man bei der Verwendung von Zusätzen wie dem in GB-A 1 347 647 und JP 0 505 898 2 A beschriebenen Imidazol, den in GB-A 1 458 747 beschriebenen Sulfonsäuren und deren Estern oder den in US-A 4 996 351 beschriebenen saurem Material, den in GB-A 1 459 691 beschriebenen Diethylsulfat und der auch dort beschriebenen Schwefelsäure sowie der Verwendung von anderen Zusätzen, wie zum Beispiel Epoxyverbindungen (DE-A 22 49 375; JP 0 932 396 8 A2), tetrasubstituierten Harnstoffen (DD 288 598), Ameisen- oder Essigsäure oder deren Derivaten (US-A 3 799 963) oder den in EP-A 524 507 beschriebenen Trimethylsilylgruppen aufweisenden Verbindungen. Der Einsatz von organischen Basen bzw. Säuren führt besonders bei der Reinigung von reaktiven Isocyanatverbindungen zu unerwünschten Nebenreaktionen wie Trimerisierung, Dimerenbildung, bzw. Carbodiimidbildung.

Einige Verbindungen mit mindestens einer Zerevitinov-aktiven NH-Gruppe wie Harnstoffe (DD 285 594), Biurete (DD 288 597), Caprolactam (DD 285 593), sowie Ammoniumsalze (DD 288 594), Carbodiimide (DD 288 599), primäre und sekundäre Aminsalze (DD 288 593), tertiäre Alkohole und tertiäre Alkylcarbamate (DD 288 595) werden nach dem Stand der Technik zur Reinigung von Isocyanaten empfohlen. Auch hier ist die Abtrennung der Zusätze bzw. die eingeschränkte Verwendung der zusatzhaltigen Isocyanate und/oder Destillationsrückstände, sowie besonders die teilweise starke Absenkung des NCO-Wertes und die Steigerung in der Viskosität, die sich zum Beispiel auf die Bildung von Biureten bei der Verwendung von tertiären Alkoholen zurückführen läßt, nachteilig. Letzteres gilt insbesondere auch für die Verwendung von Wasser zur Reinigung von Isocyanaten (DE-A 12 40 849, DE-A 1 240 849).

Die Patentschrift DD 288 596 beschreibt als Zusätze gegenüber Isocyanaten weitgehend inerte, niedermolekulare Alkylphosphate, die bei Temperaturen von mindestens 200°C unter gleichzeitigem Einsatz von Inertgasen eine effektive Absenkung des hydrolisierbaren Chlorgehaltes erlauben. In den Ausführungsbeispielen sind für eine effektive HC-Absenkung Temperaturen von 225°C für mehrere Stunden als notwendig vorgegeben. Diese hohen Temperaturen und Temperatur/Zeit-Belastungen beschränken die Anwendung des Verfahrens auf wenige relativ thermostabile Isocyanate. Isocyanatverbindungen, insbesondere temperaturempfindliche niedermolekulare Isocyanate sind bei derartig hohen Temperatur/Zeitbelastungen nicht stabil und zersetzen sich beispielsweise unter Bildung von Carbodiimiden und Isocyanuraten. Dieses kann zu einem unkontrollierten Auftreten von Prozeßwärme führen, das die Reaktionsführung erheblich erschwert. Weiterhin führt der nach DD 288 596 zwingende Einsatz von Inertgas zwangsläufig zu einem hochbelasteten Abgasstrom, der gehandhabt werden muß. Schließlich ist eine Abtrennung der beschriebenen niedermolekularen Alkylphosphate von gereinigten ebenfalls niedermolekularen Isocyanaten mittels Destillation oder Extraktion nur schwierig möglich.

Es war daher die Aufgabe der vorliegenden Erfindung, ein universell anwendbares Verfahren zur Reinigung von organischen niedermolekularen Isocyanaten, insbesondere temperaturempfindlichen niedermolekularen Isocyanaten zur Verfügung zu stellen, welches die angesprochenen Mängel nicht aufweist und eine effiziente Abtrennung des Isocyanates von den verwendeten Reagentien zur Absenkung des Anteils an hydrolysierbaren Chlor ermöglicht.

Diese Aufgabe konnte mit dem nachstehend näher beschriebenen erfindungsgemäßen Verfahren gelöst werden, bei dem den zu behandelnden organischen Isocyanaten oder Isocyanatgemischen eine geeignete Menge an nachfolgend näher beschriebenen hochmolekularen Verbindungen gemäß Formel (1) zugesetzt wird und das Gemisch für eine bestimmte Zeit, gegebenenfalls unter erhöhtem oder vermindertem Druck, erhitzt wird. Überraschenderweise erlaubt das erfindungsgernäße Verfahren eine effektive Abtrennung von störenden Chlorverbindungen (Absenkung des hydrolisierbaren Chlorgehalts) bei Temperaturen von weit unter 200°C ohne zwingenden Einsatz von Inertgas.

Gegenstand der Erfindung ist ein Verfahren zur Reinigung niedermolekularer Isocyanate dadurch gekennzeichnet, daß man die niedermolekularen Isocyanate
a) mit mindestens 0,1 % (bezogen auf Isocyanat) an Verbindungen der allgemeinen Formel (1) oder Gemischen der allgemeinen Formel (1) 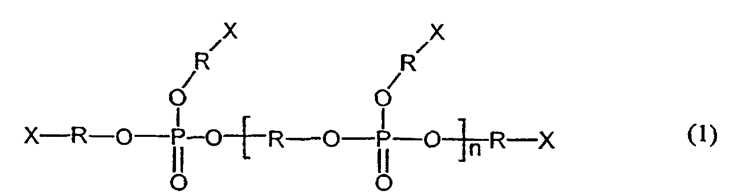 in welcher
   - n: für eine Zahl von 1 bis 20
   und
   - R: unabhängig voneinander jeweils für lineare oder verzweigte, gesättigte oder ungesättigte Alkylen-, Cycloalkylen-, Aralkylen- oder bifunktionelle Arylreste mit 1 bis 20 Kohlenstoffatomen steht, die optional Heteroatome wie z.B. Sauerstoff, Schwefel, Silicium oder Stickstoff innerhalb der Kohlenstoffkette enthalten können und/oder Sauerstoff, Schwefel, Halogenatome oder Stickstoffsubstituenten tragen können und/oder andere funktionelle Gruppen enthalten können
   und
   - X: unabhängig voneinander jeweils einen der folgenden Reste darstellen kann:
   - X: = H, OH, SH, O-R¹, S-R¹, O-Si(R¹)₃, F, Cl, Br, I, 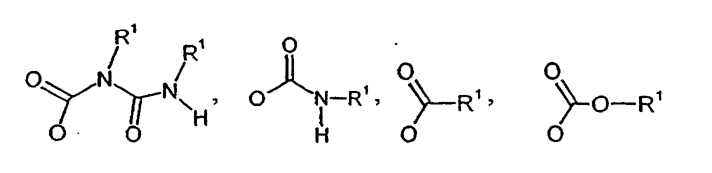
   und
   - R¹: unabhängig voneinander jeweils für Alkyl-, Cycloalkyl-, Aryl-, Aralkylreste mit 1 bis 20 Kohlenstoffatomen, die Heteroatome wie Sauerstoff, Schwefel, Silicium oder Stickstoff haben können, oder für C₁-C₂₀-Alkylen-, Cycloalkylen-, Aralkylen- oder bifunktionelle Arylreste steht, die optional Heteroatome wie Sauerstoff, Schwefel, Silicium oder Stickstoff haben können und endständig funktionelle Gruppen wie z.B. NCO haben können,
   versetzt und mit oder ohne ein inertes, nicht mit Isocyanaten reagierendes Lösungsmittel
b) für mindestens 10 Minuten auf
c) eine Temperatur von <200°C erhitzt und im Anschluß
d) gegebenenfalls mittels destillativer oder extraktiver Aufarbeitung von den hochmolekularen Verbindungen gemäß Formel (1) und/oder hochmolekularen Reaktionsprodukten befreit.

Die Verbindungen der Formel (1) können technische Produkte sein, die selbstverständlich, wenn auch weniger bevorzugt, auch noch Anteile von endständigen Säurefunktionen enthalten können. Herstellungsbedingt weisen Verbindungen der Formel (1) eine mehr oder weniger stark ausgeprägte statistische Polymerverteilung auf, so daß auch geringe Mengen an niedermolekularen Alkylphosphaten und anderen Verunreinigungen enthalten sein können.

Die nach dem erfindungsgemäßen Verfahren gereinigten, organischen Isocyanate eignen sich zur Herstellung von Beschichtungen, Polyurethanformteilen und als Zwischenprodukte.

Ausgangsmaterialien für das erfindungsgemäße Verfahren sind niedermolekulare Isocyanate, wobei hierunter auch beliebige Gemische von niedermolekularen Isocyanaten verstanden werden. Beispiele für derartige niedermolekulare Isocyanate sind
a) Monoisocyanate mit aliphatisch, cycloaliphatisch, araliphatisch oder aromatisch gebundenen Isocyanatgruppen wie z.B. Butylisocyanat, Stearylisocyanat, Cyclohexylisocyanat, Benzylisocyanat, 2-Phenylethylisocyanat, Phenylisocyanat oder Gemische solcher Monoisocyanate;
b) Diisocyanate des Molekulargewichtsbereichs 140 g/mol bis 400 g/mol mit aliphatisch, cycloaliphatisch, araliphatisch und/oder aromatisch gebundenen Isocyanatgruppen, wie z.B. 1,4-Diisocyanatobutan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat IPDI), 4,4'-Diisocyanatodicyclohexylmethan, 1-Isocyanato-1-methyl-4(3)isocyanatomethylcyctohexan (IMCI), 2,4- oder 2,6-Diisocyanato-1-methylcyclohexan (H₆-TDI), Bis-(isocyanatomethyl)-norbornan, 2-Methylpentan-2,4-diisocyanat, 1,3-und 1,4-Bis-(2-isocyanatoprop-2-yl)-benzol (TMXDI), 2,4- und 2,6-Diisocyanatotoluol (TDI), 2,4'- und 4,4'-Diisocyanatodiphenylmethan, 1,5-Diisocyanatonaphthalin, Dipropylenglycoldiisocyanat oder beliebige Gemische solcher Diisocyanate,
c) Triisocyanate und/oder höherfunktioneller Isocyanate wie z.B. 4-Isocyanatomethyl-1,8-octandiisocyanat (Nonantriisocyanat), 1,6,11 -Undecantriisocyanat oder beliebige Gemische solcher Isocyanate.

Ausgangsmaterialien für das erfindungsgemäße Verfahren können selbstverständlich auch beliebige Gemische aus Mono- und/oder Di- und/oder höherfunktionellen Triisocyanaten sein.

Allgemein können als niedermolekulare Isocyanate alle organischen Isocyanate eines Molekulargewichts bis ca. 400 g/mol, vorzugsweise von 99 bis 279 g/mol Verwendung finden.

Bevorzugt kommen beim erfindungsgemäßen Verfahren die genannten Di- und höherfünktionellen Isocyanate zum Einsatz. Ganz besonders bevorzugt ist die Verwendung von 4-Isocyanatomethyl-1,8-octandiisocyanat (Nonantriisocyanat).

Das erfindungsgemäße Verfahren sieht vor, das Gemisch aus organischem Isocyanat und der Verbindung gemäß Formel (1) mit mindestens 0,1 %, vorzugsweise 0,5 bis 50 % und besonders bevorzugt 1,0 bis 5 % bezogen auf Masse Isocyanat für mindestens 10 Minuten, vorzugsweise 1 Stunde bis 24 Stunden, besonders bevorzugt 3 Stunden bis 15 Stunden bei einer Temperatur von <200°C, vorzugsweise 140°C bis 190°C und besonders bevorzugt 150°C bis 180°C, mit oder bevorzugt ohne ein inertes nicht mit Isocyanaten reagierendes Lösungsmittel zu erhitzen und gegebenenfalls im Anschluß das gereinigte, niedermolekulare Isocyanat von den hochmolekularen Verbindungen gemäß Formel (1) und/oder hochmolekularen Reaktionsprodukten sowie dem optional vorhandenen inerten, nicht mit Isocyanaten reagierenden Lösungsmitteln destillativ oder extraktiv zu befreien.

Das Isocyanat kann während oder nach der Temperung extraktiv oder destillativ, gegebenenfalls im Vakuum, von höhermolekularen Verbindungen entfernt werden. Vorzugsweise wird das gereinigte Isocyanat unter Vakuum (z.B. 0.001 mbar bis 100 mbar) von den hochmolekularen Verbindungen gemäß Formel (1) und/oder hochmolekularen Reaktionsprodukten, destillativ entfernt.

Zur Vermeidung von Nebenreaktionen mit Luft bzw. Spuren von Wasser kann die Reaktionsapparatur unter Vakuum (beispielsweise 100 bis 1 mbar) betrieben werden oder eine Beschleierung mit Inertgas, beispielsweise Stickstoff, erfolgen. Prinzipiell ist der Einsatz von Inertgas für das erfindungsgemäße Verfahren jedoch nicht zwingend notwendig.

Üblicherweise haben die nach dem erfindungsgemäßen Verfahren erhaltenen Isocyanate Gehalte an hydrolysierbaren Chlor von <400 ppm, bevorzugt <250 ppm und besonders bevorzugt <150 ppm.

Vor, während oder nach der Absenkung des Chlorgehaltes nach dem erfindungsgemäßen Verfahren können auch noch andere Reinigungsmethoden Verwendung finden, um beispielsweise farbgebende Komponenten und Nebenprodukte zu entfernen. Hierzu zählen unter anderem Behandlungen und/oder Aufhellungen beispielsweise mit Reduktions- oder Oxidationsmitteln und die Behandlung mit Adsorptionsmitteln wie Aktivkohle und/oder Kieselsäuren. Derartige Aufhellungen können ebenfalls einen positiven Effekt auf die Absenkung des Chlorgehalts der Isocyanatverbindung ausüben.

Erfindungsgemäß gereinigte Isocyanate weisen keine schädlichen Zusätze von Metallverbindungen, Säuren, Basen oder anderen isocyanatreaktiven Verbindungen auf und haben einen HC-Gehalt von vorzugsweise <250 ppm. Sie können breit eingesetzt werden z.B. zur Herstellung von oligomeren Polyisocyanaten oder Prepolymeren und im Falle der Triisocyanate als Ausgangsmaterialien für Zwischenprodukte, Polyurethanformteile und Beschichtungsmittel. Vorzugsweise werden die nach dem erfindungsgemäßen Verfahren gereinigten niedermolekularen Triisocyanate als Härterkomponente in Beschichtungen eingesetzt.

Prinzipiell eignen sich Beschichtungsmaterialien, die nach dem erfindungsgemäßen Verfahren gereinigte Isocyanate enthalten, zur Beschichtung beliebiger Substrate wie beispielsweise Holz, Kunststoffe, Leder, Papier, Textilien, Glas, Keramik, Putz, Mauerwerk, Metalle oder Beton. Sie lassen sich mit üblichen Applikationsmethoden wie Spritzen, Streichen, Fluten, Gießen, Tauchen, Walzen aufbringen. Die Beschichtungsmaterialien können in Form von Klarlacken als auch in Form pigmentierter Lacke verwendet werden, wobei sie verdünnt in organischen Lösemitteln bzw. dispergiert in Wasser oder unverdünnt als Ein- oder Mehrkomponentenbeschichtung zum Einsatz gelangen.

### Beispiele

Die angegebenen HC-Werte beziehen sich auf den Gehalt an hydrolisierbarem Chlor. Alle Prozentangaben beziehen sich auf das Gewicht.

100 g des organischen Isocyanates werden mit der in Tabelle 1 bis 2 angegebenen Menge an den Verbindungen A oder B versetzt und unter Vakuum (10 bis 100 mbar; je nach eingesetztem Isocyanat) für die angegebene Zeitspanne und Temperatur gerührt. Nach der angegebenen Reaktionszeit werden die Produkte einer dünnschichtdestillativen Aufarbeitung (180°C/0,2 mbar) unterzogen. Man erhält die erfindungsgemäßen Produkte.

**Tabelle 1**

| | Vergleichsbeispiel 1 | VergleichsBeispiel 2 Entsprechend DD 288 596 1. Beispiel | erfindungsgemäßes Beispiel 1 | erfindungsgemäßes Beispiel 2 | erfindungsgemäßes Beispiel 3 |
|---|---|---|---|---|---|
| Organisches Isocyanat | Triisocyanatononan (TIN) | Triisocyanato-Nonan (TIN) | Triisocyanatononan (TIN) | Triisocyanatononan (TIN) | Triisocyanatononan (TIN) |
| Verwendeter Zusatz | - | Triethylphosphat | A | B | A |
| Gew.-% des Zusatzes | - | 2,5 | 2,5 | 2,5 | 10 |
| Temperatur | 170°C | 225°C | 160°C | 170°C | 170°C |
| Reaktionszeit (h) | 12 | 3 | 24 | 12 | 12 |
| HC-Wert vor Konditionierung (ppm) | 5000 | 5000 | 5000 | 5000 | 5000 |
| HC-Wert nach Kondtionierung (ppm) | 1500 | <100 | <100 | <100 | <100 |
| NCO vor Temperung (%) | 47,1 | 44,7 | 46,0 | 46,0 | 46,0 |
| NCO nach Temperung (%) | 44,2 | 37,5 | 41,2 | 42,0 | 41,1 |
| Verbindung A: Exolit OP 550 (Fa. Clariant) (OH-Zahl: 130; Säurezahl <1, Dichte: 1,31 g/cm³ bei 25°C, Viskosität: ca. 2000 mPas) | | | | | |
| Verbindung B: Umsetzungsprodukt aus 1,0 eq. Exolit OP 550 (Fa. Clariant) und 0,9 eq. Butylisocyanat | | | | | |

Der Unterschied des NCO-Gehalts vor und nach der Temperung gibt Auskunft über unerwünschte Nebenreaktionen und somit den Ausbeuteverlust.

Im nicht erfindungsgemäßen Vergleichsbeispiel 2 werden die Bedingungen der Patentschrift DD 288 596 nachgestellt, um den HC-Gehalt von TIN abzusenken. Die deutliche NCO-Abnahme macht auf eine nicht akzeptabel hohe Belastung aufmerksam. Die Differenzthermoanalyse zeigt bei dieser Temperatur eine starke, zusätzliche unkontrollierte Wänneproduktion der Reaktion (>600 kJ/kg), die im technischen Verfahren nicht tragbar ist.

### Beispiel 2 (erfindungsgemäßes Verfahren):

Schon bei 170°C kann der HC-Gehalt bei schonenden Bedingungen auf unter 100 ppm abgesenkt werden, wobei nur eine geringe NCO-Abnahme festzustellen ist.

**Tabelle 2**

| | Vergleichsbeispiel 3 | erfmdungsgemäßes Beispiel 4 | erfindungsgemäßes Beispiel 5 | erfindungsgemäßes Beispiel 6 |
|---|---|---|---|---|
| Organisches Isocyanat | 4,4'-Diisocyanatodiphenylmethan | 4,4'-Diisocyanatodiphenylmethan | Isophorondiisocyanat (IPDI) | Hexarnethylendiisocyanat (HDI) |
| verwendeter Zusatz | - | B | B | B |
| Gew.-% des Zusatzes | - | 2,5 | 2,5 | 2,5 |
| Temperatur | 170°C | 170°C | 170°C | 170°C |
| Reaktionszeit (h) | 12 | 12 | 12 | 12 |
| HC-Wert vor Konditiionierung (ppm) | 391 | 391 | 156 | 48 |
| HC-Wert nach Konditionierung (ppm) | 220 | 35 | 14 | 12 |
| NCO vor Temperung (%) | 31,3 | 30,6 | 36,9 | 48,8 |
| NCO nach Temperung (%) | 31,1 | 24,6 | 35,7 | 45,8 |

## Patentansprüche

1. Verfahren zur Reinigung niedermolekularer Isocyanate **dadurch gekennzeichnet, daß** man die niedermolekularen Isocyanate
a) mit mindestens 0,1 % (bezogen auf Isocyanat) an Verbindungen der allgemeinen Formel (1) oder Gemischen der allgemeinen Formel (1) 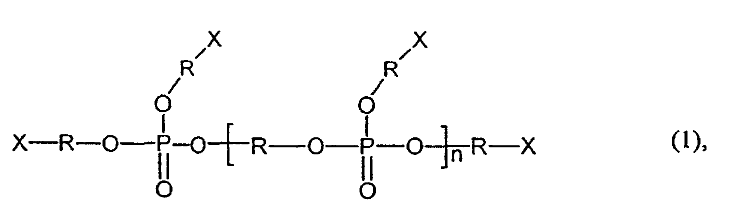 in welcher
n für eine Zahl von 1 bis 20
und
R unabhängig voneinander jeweils für lineare oder verzweigte, gesättigte oder ungesättigte Alkylen-, Cycloalkylen-, Aralkylen- oder bifunktionelle Arylreste mit 1 bis 20 Kohlenstoffatomen steht, die optional Heteroatome wie z.B. Sauerstoff, Schwefel oder Stickstoff innerhalb der Kohlenstoffkette enthalten können und/oder Sauerstoff, Schwefel, Halogenatome oder Stickstoffsubstituenten tragen können und/oder andere funktionelle Gruppen enthalten können
und
X unabhängig voneinander jeweils einen der folgenden Reste darstellen kann:
X = H, OH, SH, O-R¹, S-R¹, O-Si(R¹)₃, F, Cl, Br, I, 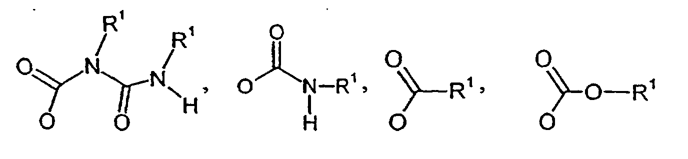
R¹ unabhängig voneinander jeweils für Alkyl-, Cycloalkyl-, Aryl-, Aralkylreste mit 1 bis 20 Kohlenstoffatomen, die Heteroatome wie Sauerstoff, Schwefel, Silicium oder Stickstoff haben können, oder für C₁-C₂₀-Alkylen-, Cycloalkylen-, Aralkylen- oder bifunktionelle Arylreste steht, die optional Heteroatome wie Sauerstoff, Schwefel, Silicium oder Stickstoff haben können und endständig funktionelle Gruppen wie z.B. NCO haben können,
versetzt und mit oder ohne ein inertes, nicht mit Isocyanaten reagierendes Lösungsmittel
b) für mindestens 10 Minuten auf
c) eine Temperatur von <200°C erhitzt und im Anschluß
d) gegebenenfalls mittels destillativer oder extraktiver Aufarbeitung von den hochmolekularen Verbindungen gemäß Formel (1) und/oder hochmolekularen Reaktionsprodukten befreit.

2. Verfahren gemäß Anspruch 1, **dadurch gekennzeichnet, daß** man die niedermolekularen Isocyanate
a) mit 1,0 bis 5,0 Gew.-% bezogen auf Isocyanat an den hochmolekularen Verbindungen gemäß der allgemeinen Formel (1) versetzt
b) für 3 Stunden bis 15 Stunden auf
c) eine Temperatur von 150 bis 180°C erhitzt und im Anschluß
d) mittels destillativer Aufarbeitung von den hochmolekularen Verbindungen gemäß Formel (1) und/oder hochmolekularen Verbindungen gemäß Formel (1) und/oder hochmolekularen Reaktionsprodukten befreit.

3. Verfahren gemäß Anspruch 1 und 2, **dadurch gekennzeichnet, daß** man als organisches Isocyanat 4-Isocyanatomethyl-1,8-octandiisocyanat (Nonantriisocyanat) verwendet.

4. Verfahren gemäß Anspruch 1, **dadurch gekennzeichnet, daß** die so gereinigten Isocyanate einen Gehalt an hydrolysierbarem Chlor von <250 ppm aufweisen.

## Claims

1. Process for purifying low molecular weight isocyanates, **characterized in that** the low molecular weight isocyanates
a) are admixed with at least 0.1% (based on isocyanate) of compounds of the general formula (1) or mixtures of the general formula (1) 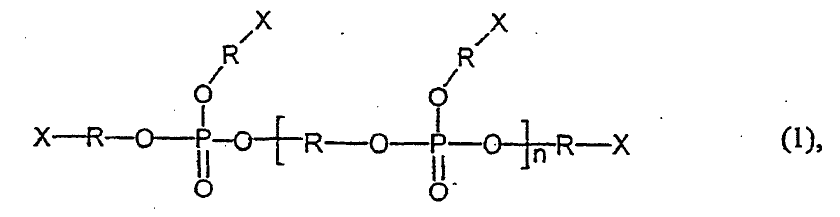 in which
n is a number from 1 to 20
and
R independently at each occurrence is a linear or branched, saturated or unsaturated alkylene, cycloalkylene, aralkylene or bifunctional aryl radical having 1 to 20 carbon atoms which may optionally contain heteroatoms such as oxygen, sulphur or nitrogen, for example, within the carbon chain and/or may carry oxygen, sulphur, halogen atoms or nitrogen substituents and/or may contain other functional groups
and
X independently at each occurrence can be one of the following radicals:
X = H, OH, SH, O-R¹, S-R¹, O-Si(R¹)₃, F, Cl, Br, I, 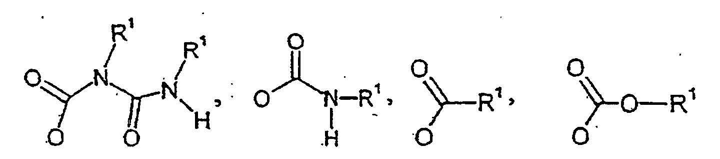
R¹ independently at each occurrence is an alkyl, cycloalkyl, aryl or aralkyl radical having 1 to 20 carbon atoms which may have heteroatoms such as oxygen, sulphur, silicon or nitrogen or is a C₁-C₂₀ alkylene, cycloalkylene, aralkylene or bifunctional aryl radical which may optionally have heteroatoms such as oxygen, sulphur, silicon or nitrogen and may terminally have functional groups such as NCO, for example,
and heated with or without an inert solvent which is not reactive with isocyanates
b) for at least 10 minutes at
c) a temperature of <200°C and subsequently
d) optionally by means of distillative or extractive workup are freed from the high molecular weight compounds of formula (1) and/or high molecular weight reaction products.

2. Process according to Claim 1, **characterized in that** the low molecular weight isocyanates
a) are admixed with from 1.0 to 5.0% by weight, based on isocyanate, of the high molecular mass compounds of the general formula (1),
b) heated for from 3 hours to 15 hours at
c) a temperature of from 150 to 180°C and subsequently
d) by means of distillative workup freed from the high molecular weight compounds of formula (1) and/or high molecular weight reaction products.

3. Process according to Claim 1 and 2, **characterized in that** 4-isocyanatomethyl-1,8-octane diisocyanate (nonane triisocyanate) is used as organic isocyanate.

4. Process according to Claim 1, **characterized in that** the isocyanates thus purified have a hydrolysable chlorine content of <250 ppm.

## Revendications

1. Procédé pour la purification des isocyanates à bas poids moléculaire, **caractérisé en ce que**
a) on ajoute aux isocyanates à bas poids moléculaire au moins 0,1 % (par rapport aux isocyanates) de composés de formule générale (1) ou de mélanges de formule générale (1) 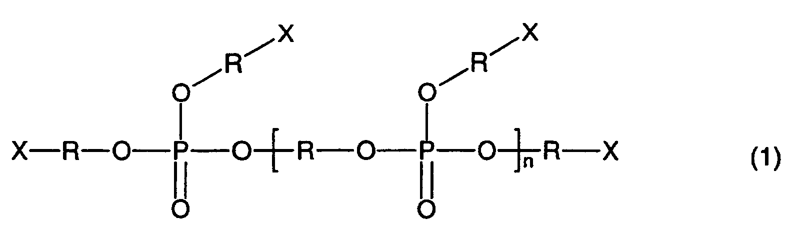 dans laquelle
n est un nombre allant de 1 à 20,
les symboles R représentent chacun, indépendamment les uns des autres, un groupe alkylène à chaîne droite ou ramifiée, saturé ou insaturé, cycloalkylène, aralkylène ou aryle bifonctionnel en C₁ à C₂₀, qui peuvent contenir éventuellement des hétéroatomes tels que l'oxygène, le soufre ou l'azote à l'intérieur de la chaîne carbonée et/ou peuvent porter de l'oxygène, du soufre, des atomes d'halogènes ou des substituants azotés et/ou peuvent contenir d'autres groupes fonctionnels,
les symboles X ont chacun, indépendamment les uns des autres, l'une des significations suivantes :
X = H, OH, SH, O-R¹, S-R¹, O-Si(R¹)₃, F, Cl, Br, I, 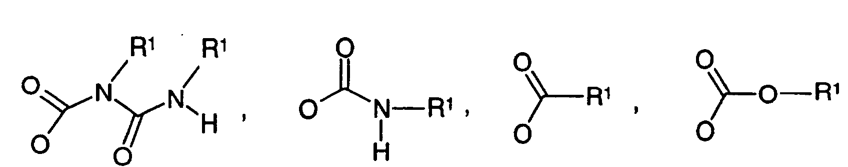
les symboles R¹ représentent chacun, indépendamment les uns des autres, un groupe alkyle, cycloalkyle, aryle, aralkyle en C₁ à C₂₀, qui peuvent contenir des hétéroatomes tels que l'oxygène, le soufre, le silicium ou l'azote, ou un groupe alkylène en C₁ à C₂₀, cycloalkylène, aralkylène ou aryle bifonctionnel, qui peuvent contenir facultativement des hétéroatomes tels que l'oxygène, le soufre, le silicium ou l'azote et peuvent porter dans les positions terminales des groupes fonctionnels, par exemple NCO,
et en présence ou non d'un solvant inerte qui ne réagit pas avec les isocyanates,
b) on chauffe pendant au moins 10 min
c) à une température inférieure à 200°C, puis
d) le cas échéant, on les débarrasse des composés à haut poids moléculaire de formule (1) et/ou des produits de réaction à haut poids moléculaire par un traitement de distillation ou d'extraction.

2. Procédé selon la revendication 1, **caractérisé en ce que**
a) on ajoute aux isocyanates à bas poids moléculaire 1,0 à 5,0 % de leur poids des composés à haut poids moléculaire de formule générale (1),
b) on chauffe pendant 3 à 15 h
c) à une température de 150 à 180°C, puis
d) on les débarrasse des composés à haut poids moléculaire de formule (1) et/ou des produits de réaction à haut poids moléculaire par un traitement de distillation.

3. Procédé selon les revendications 1 et 2, **caractérisé en ce que** l'isocyanate organique soumis au traitement est le 4-isocyanatométhyl-1,8-octanediisocyanate (nonanetriisocyanate).

4. Procédé selon la revendication 1, **caractérisé en ce que** les isocyanates purifiés ont une teneur en chlore hydrolysable inférieure à 250 ppm.
